# EUROPÄISCHE PATENTSCHRIFT

(11) **EP 1 084 156 B1**
(45) Veröffentlichungstag und Bekanntmachung des Hinweises auf die Patenterteilung: **06.10.2004**
(21) Anmeldenummer: 99917936.9
(22) Anmeldetag: 31.03.1999
(51) Int. Cl.: C08F 6/00

(54) **VERFAHREN ZUR VERMINDERUNG DES -C1-C-2-ALDEHYD-GEHALTS AUS VERBINDUNGEN MIT -CH2-CHR-O- ODER -CH2-CH(OH)-GRUPPEN**
METHOD FOR REDUCING THE C1-C2-ALDEHYDE CONTENT FORMED FROM COMPOUNDS WITH -CH2-CHR-O- OR -CH2-CH(OH)-GROUPS
PROCEDE POUR REDUIRE LA TENEUR EN ALDEHYDES C1-C2 DANS DES COMPOSES A GROUPES -CH2-CHR-O- OU -CH2-CH(OH)

(30) Priorität: 02.04.1998 DE 19814873
(43) Veröffentlichungstag der Anmeldung: 21.03.2001
(73) Patentinhaber: BASF AKTIENGESELLSCHAFT, 67056 Ludwigshafen (DE)
(72) Erfinder: RUPANER, Robert, CEP-12500-000 Guaratingueta, SP (BR); SCHOLTISSEK, Martin, D-68165 Mannheim (DE); SCHUMACHER, Karl-Heinz, D-67433 Neustadt (DE); ANGEL, Maximilian, D-67105 Schifferstadt (DE)
(74) Vertreter: Kinzebach, Werner, Dr.
(86) Internationale Anmeldenummer: PCT/EP1999/002216
(87) Internationale Veröffentlichungsnummer: WO 1999/051647

(56) Entgegenhaltungen:
- EP-A- 0 080 635
- EP-A- 0 143 175
- EP-A- 0 647 658
- EP-A- 0 661 305

## Beschreibung

Die Erfindung betrifft ein Verfahren zur Verminderung eines unter Radikaleinwirkung aus Verbindungen mit -CH₂-CHR-O- oder CH₂-CH(OH)-Gruppierungen gebildeten Gehalts an C₁-C₂-Aldehyden.

Es ist bekannt, daß wässrige Polymerdispersionen oder -lösungen von ihrer Herstellung her Aldehydderivat-Gruppierungen von Monomeren, Vernetzern oder Initiatoren oder deren Komponenten enthalten können, z.B. N-Methylol-Gruppen, veresterte oder veretherte N-Methylol-Gruppen, die bestimmte Mengen an C₁-C₂-Aldehyden wie Formaldehyd bei deren Lagerung oder Verwendung oder in Gegenwart von Säure freisetzen können. Die entsprechenden Dispersionen oder Lösungen finden aufgrund ihres Gehalts an freiem Formaldehyd für eine ganze Reihe von Produkten, wie für Sanitärprodukte, keine Anwendung. Es ist bekannt, solchen aldehydabspaltenden Produkten Aldehydfänger zuzusetzen, die in der Lage sind, den freigesetzten Aldehyd und insbesondere den Formaldehyd zu binden und damit die Produkte aus toxikologischer Sicht akzeptabler zu machen (siehe z.B. EP-A 80635, EP-B 143175, EP-B 492378, EP-A 527411). Es ist auch bekannt (EP-A 492 378, EP-A 505959), unerwünschte Mengen an Formaldehyd oder Acetaldehyd durch Oxydationsreaktionen zu entfernen.

Es wurde nun gefunden, daß nicht nur wässrige Polymerdispersionen oder -lösungen, die Aldehydderivat-Gruppierungen der genannten Art enthalten, C₁-C₂-Aldehyde wie Formaldehyd freisetzen können, sondern daß dies auch z.B. wässrige Polymerdispersionen und -lösungen können, die als Emulgatoren oder Schutzkolloide Verbindungen mit -CH₂-CHR-O- oder -CH₂-CH(OH)-Gruppierungen im Molekül enthalten (worin R = H oder C₁-C₄-Alkylrest ist), wenn sie, z.B. bei ihrer Herstellung einer Radikaleinwirkung ausgesetzt wurden. Verbindungen mit den genannten Gruppierungen sind z.B. nicht-ionogene ethoxylierte C₆-C₂₀-Alkohole, C₆-C₂₀-Amine oder C₄-C₁₈-Alkylphenole mit einem Ethoxylierungsgrad von 1 bis 100 Mol Ethylenoxid, Polyethylenoxid mit 30 bis 8000 Mol Ethylenoxid im Molekül, Blockcopolymere von Polyethylenoxid und Polypropylenoxid oder Polyvinylalkohole wie Polyvinylester, deren Estergruppen (z.B. Acetatgruppen) zu 50 bis 98 Mol% verseift wurden. Die ethoxylierten Alkohole, Amine oder Alkylphenole können dabei auch in ionogener, z.B. sulfatierter oder phosphonierter Form oder in Salzform davon vorliegen. Weitere ethoxylierte Verbindungen mit Polyetherketten, die bei der Herstellung von Polymerdispersionen Verwendung finden, sind z.B. ethoxylierte Hydroxyethylcellulosen, ethoxylierte Stärkederivate und ethoxylierte Monomere, wie ethoxyliertes 2-Hydroxyethylmethacrylat. Einen Überblick über so strukturierte Verbindungen, die bei der Polymerisation Verwendung finden können, geben z.B. N.Schönfeld, Grenzflächenaktive Äthylenoxid-Addukte, Stuttgart 1976 und der Ergänzungsband von 1984, sowie Houbenweyl, Bd. XIV/1, Stuttgart 1961, Seiten 190-208. Diese Verbindungen mit -CH₂-CHR-O- und/oder -CH₂-CH(OH)-Gruppierungen, die zum großen Teil seit Jahrzehnten industriell bei der Produktion wässriger Dispersionen und Lösungen verwendet werden, galten lange Zeit als gesundheitlich gänzlich unbedenklich, da deren Aldehyd-Abspaltung von anderen Aldehyd-abspaltenden Reaktionen verdeckt wurde. In der Literatur ist zwar angegeben, daß energiereiche Strahlen, Sauerstoff oder Peroxide bei Polyethylenoxid- oder Polypropylenoxidketten eine Vielzahl von Reaktionen bewirken können, die zu einem Abbau oder in-folge von Vernetzungsreaktionen auch zu einer Vergrößerung des Molekulargewichts führen können (siehe z.B. Kaczmarek et al., Macromol.Symp.84(1994) 351-363; Janik, Abh.Akad. Wissenschaften DDR, Akademieverlag Berlin 1987, 551-555; Hidata et al. Yukagaku 1990, 39 (11), 963-966, C.A. 114: 8577m). Daß solche Produkte bei Radikaleinwirkung aber in Spuren Formaldehyd, Acetaldehyd und /oder Glyoxal freisetzen können, war mit seinen Folgen nicht erkannt.

Zur Erklärung des Abbaus solcher Gruppierungen sei darauf hingewiesen, daß in einer Etherkette H-Atome einer CHₓ-O- Gruppe durch Radikale leicht abstrahiert werden können. Nach dem H-Transfer liegt das Radikalzentrum an der Ethylenoxid-Kette, die nun Folgereaktionen eingehen kann, wie eine Monomeranlagerung (Emulgatorpfropfung), eine Kombination mit anderen Radikalen oder ein Abbau der Polyetherkette. Letzterer führt zur Bildung niedermolekularer Verbindungen. Stets, wenn eine -CHₓ-O-Verbindung mit einem Radikal oder einem Radikalbildungsmechanismus zusammentrifft, kann ein radikalisch-induzierter Abbau erfolgen, auch bei Ethylenglykol, Ethylenglykoldimethylether oder Polyvinylalkohol. Daß bei einem solchen radikalisch induziertem Abbau deutliche Mengen an Formaldehyd, Acetaldehyd und/oder Glyoxal gebildet werden können, ist eine neue Erkenntnis.

Da Formaldehyd, Acetaldehyd und/oder Glyoxal auch in geringen Konzentrationen in Produkten unerwünscht sind, zu Verfärbungen der Produkte führen können und insbesondere eine Präsenz von Formaldehyd in Produkten zu toxikologischen Vorbehalten beim Verarbeiter führt, stellte sich die neue Aufgabe, in Substanzen, Dispersionen oder Lösungen, die CH₂-CHR-O- (R = H,C₁-C₄-Alkyl) oder -CH₂-CH(OH)-Gruppen enthalten oder Verbindungen mit solchen Gruppen enthalten, den bei Radikaleinwirkung entstehenden Gehalt an kleinen Mengen an C₁-C₂-Aldehyden zu vermindern, möglichst auf Mengen unter 1 ppm. Dieser Aufgabe kommt auf dem Gebiet der Verwendung von Kunststoffdispersionen besondere Bedeutung zu, da z.B. für die Herstellung von Vliesstoffen für den Sanitär- und Hygienebereich in Teilsektoren der Anwendung die Auflage besteht, nur formaldehydfreie Kunststoffdispersionen zu verwenden (vgl. EP-A 143175,Seite 2, Zeile 55 ff.).

Es wurde nun gefunden, daß diese neue Aufgabe gelöst werden kann mit einem Verfahren zur Verminderung eines unter Radikaleinwirkung aus organischen Verbindungen mit -CH₂-CHR-O- oder CH₂-CH(OH)-Gruppierungen (R = H, C₁-C₄-Alkyl) gebildeten Gehalts an C₁-C₂-Aldehyden in Substanzen, Dispersionen oder Lösungen, die solche Gruppierungen oder Verbindungen mit solchen Gruppierungen enthalten, die keine zugesetzten Verbindungen mit Aldehydderivat-Gruppen aufweisen, die bekannterweise C₁-C₂-Aldehyde freisetzen können, durch Zusatz von mit den Aldehyd-Gruppen reagierenden stickstoffhaltigen Verbindungen, welche den Aldehyd binden und die ausgewählt sind unter Harnstoff; Harnstoff-Derivaten, auch cyclischen Harnstoffderivaten; Copolymeren, die Monomere mit cyclischen Harnstoffgruppen enthalten; wasserlöslichen Copolymeren mit Amidgruppen; Carbamaten und cyclischen Verbindungen mit NH-Gruppen. Es war überraschend, daß so der Gehalt an C₁-C₂-Aldehyden auf Mengen von 1 ppm und darunter, d.h. unter die Nachweisgrenze vermindert werden kann.

Für die Durchführung der Verminderung des C₁-C₂-Aldehydgehalts durch Zusatz von Verbindungen, die mit Aldehydgruppen reagieren, wie Aldehydfängern, können praktisch die gleichen bekannten Verfahren angewandt werden, die zur Aldehydverminderung bei Systemen mit Aldehydderivaten im Polymeren wie Copolymeren mit ggf. veretherten oder veresterten N-Methylolacrylamidgruppen bekannt sind und vielfach auf den Gebieten der Lack- und der Textilchemie Anwendung finden. So beschreibt H.Petersen, Textiles Res.J. 51(1981) 282-301 oder R.S.Perry et al., Textile Chemists and Colorists Vol. 12(1980) 311-316 die Absenkung von Formaldehyd mit verschiedenen Formaldehydfängern. Harnstoff als Formaldehydfänger ist in der EP-A 80635 beschrieben, cyclischer Ethylenharnstoff (2-Imidazolidon) und Propylenharnstoff in der EP-A 143175 oder der GB 2086929. Wasserlösliche Copolymere mit Amidgruppen werden in der EP-A 527411 zur Absenkung des Formaldehyd-Gehalts verwendet, auch Copolymere, die einpolymerisierte Monomere mit cylischen Harnstoffgruppen enthalten, eignen sich dafür (EP-A 488605). Maßnahmen zur Absenkung des Formaldehydgehalts wurden und werden auch vielfach auf dem Gebiet der Spanplattenverarbeitung angewandt (vgl.GB 2086929, EP-A 341569) und insbesondere zur Entfernung von Formaldehyd aus mit wässrigen Polymerdispersionen oder wässrigen Lösungen von Aminoplast-Kondensaten behandelten (imprägnierten) Textilien (US-A 3590 100, US-A 3957431).

Neben Harnstoff und Carbamaten werden auch cyclische Verbindungen mit NH-Gruppen wie Verbindungen vom Pyrrolidon-Typ, ferner Benzimidazolverbindungen (US-A 4127 382) als Formaldehydfänger benutzt .

Bei neuer Aufgabe und bei anderen Ausgangsprodukten ist die durchzuführende Maßnahme, C₁-C₂-Aldehyde durch Zusätze zu binden oder chemisch abzuwandeln, im Prinzip an sich bekannt, und sie kann somit gemäß den Angaben im Stand der Technik ausgeführt werden. Die verfahrensgemäß zu behandelnden Substanzen, Dispersionen oder Lösungen sind oder enthalten stets organische Verbindungen mit -CH₂-CHR-O- (worin R= H oder C₁-C₄-Alkyl) und/oder -CH₂-CH(OH)-Gruppierungen, aus denen sich bei Radikaleinwirkung wie bei einem Zerfall von Radikalinitiatoren kleine, aber störende Mengen an C₁-C₂-Aldehyden bilden können. Substanzen mit den genannten Gruppierungen sind z.B. Polyvinylalkohole, Alkylenglykole mit 2 bis 4 C-Atomen, Polymere davon sowie Derivate solcher Verbindungen wie Ethylenglykoldimethylether oder Alkylenoxid- und insbesondere Ethylenoxid-Anlagerungsprodukte an z.B. langkettige Alkohole, Phenole, Amine etc., wie sie als Emulgatoren und Schutzkolloide vielfach Verwendung finden. Auf die anfangs angegebenen Beispiele solcher Verbindungen sei verwiesen. Besondere Bedeutung hat das erfindungsgemäße Verfahren bei der Behandlung von Dispersionen und Lösungen, die Verbindungen mit den genannten Gruppierungen enthalten, die als Emulgatoren und/oder Schutzkolloide bei der Herstellung und/oder Verwendung von Polymerdispersionen wirken. So ist es bedeutsam zur verfahrensgemäßen Behandlung von wässrigen Polymerdispersionen oder -lösungen, die durch radikalische Polymerisation und/oder radikalische Nachpolymerisation von olefinisch ungesättigten Monomeren in Gegenwart von organische Verbindungen mit -CH₂-CHR-O- und/oder -CH₂-CH(OH)-Gruppierungen hergestellt sind. Da das erfindungsgemäße Verfahren für die Absenkung von kleinen Mengen von durch Radikaleinwirkung aus - CH₂-CHR-O- oder -CH₂-CH(OH)-Gruppen entstandenen C₁-C₂-Aldehyden auf Gehalte von möglichst unter 1 ppm dienen soll, ist es natürlich nicht sinnvoll, in den zu behandelnden Substanzen, Dispersionen oder Lösungen, weitere Substanzen zu haben, die bekannterweise C₁-C₂-Aldehyde freisetzen können. So sollten die verfahrensgemäß zu behandelnden Substanzen, Dispersionen oder Lösungen auch keine Monomeren, Monomereinheiten, Initiatoren und /oder Vernetzer mit Aldehydderivatgruppen, wie N-Methylolgruppen, veretherten oder veresterten N-Methylolgruppen, enthalten, die bekannterweise leicht Aldehydgruppen freisetzen.

In den nachstehenden Beispielen wird gezeigt, daß Emulgatoren, Schutzkolloide und Blockcopolymere, die Ethylenoxidgruppen gebunden enthalten, in Gegenwart von Radikalen abgebaut werden und dabei C₁-C₂-Aldehyde, insbesondere Formaldehyd oder Acetaldehyd entstehen. Gleiches gilt für eine Radikaleinwirkung auf Monomerenemulsionen, die Verbindungen mit -CH₂-CHR-O- oder -CH₂-CH(OH)-Gruppen (R = H, C₁-C₄-Alkyl) im Emulgator, Schutzkolloid oder Monomerem enthalten.

Der Nachweis von Formaldehyd erfolgte wie folgt:
Methode 1: Der Nachweis von Formaldehyd neben anderen Carbonylverbindungen erfolgte durch die Kombination von HPLC-Trennung von Formaldehyd und einer chromatographischen Nachsäulenderivatisierung mit Acetylaceton, wobei das entstehende Lutidinderivat gegen eine externe Kalibrierung quantifiziert wurde. Die Methode ist u.a. von H. Engelhardt und R. Klinckner in dem Artikel "Determination of Free Formaldehyde in the Presence of Donators in Cosmetics by HPLC and Post Column Derivation" in Chromatographia Vol.20, No.9 (1985) 559-565 und von U. Schäfer-Lüdderssen und M. Mauß im Artikel "Determination of Free Formaldehyde in Nonwovens in the Presence of Glyoxalic Acid and Aldehydes" in Chromatographia vol.29, No.1/2 (1990) 21-23 beschrieben.
Methode 2: Eine Probe der zu untersuchenden Substanz wird mit 2,4-Dinitrophenylhydrazin umgesetzt und die erhaltenden Hydrazone durch HPLC an einer RP-Phase aufgetrennt (Säule Licrospher 100 RP 18,5 µm, (125x4 mm), Eluent H₂O/Acetonitril, Fluß 1,0 ml/Min., Detektion bei 370 nm). Die Identifizierung des Produktpeaks wurde mit Hilfe von Referenzsubstanzen durchgeführt.

Soweit nicht anders angegeben, beziehen sich die in den nachstehenden Beispielen angegebenen Teile und Prozente auf das Gewicht. Eine Angabe "(xEO)" bedeutet, daß x Moleküle Ethylenoxid mit einem Molekül der entsprechenden Verbindung durch Ethoxylierung verbunden wurden.

Zur Schaumbestimmung wurde durch einen skalierten Glaszylinder mit Glasfritte (Innendurchmesser 35 mm, Porosität der Fritte: 2) über ein Druckminderungsventil Stickstoff bei einem Druck von etwa 0,2 bar und 3,5 Liter/Minute durchgeblasen. Nach Einfüllen von 25 g Polymerdispersion wurde die Schaumentwicklung volumenmäßig zeitlich verfolgt und der 6 Minutenwert registriert.

Die Lichtdurchlässigkeit (LD-Wert) wurde durch Vergleich der Lichtdurchlässigkeit einer 0,01 Gew.%igen Probe der Polymerdispersion bei einer Schichtdicke von 25 mm mit der von reinem Wasser bestimmt.

Der Restmonomerengehalt wurde gaschromatographisch ermittelt. Die Feststoffgehalte (FG) in Gew.-% wurden nach dem Eintrocknen gravimetrisch bestimmt.

### Beispiel 1 - Formaldehyd aus ethoxylierte Emulgatoren

### a. Emulgatoren: Jeweils 3,2 %ige Stammlösungen wurden mit den folgenden Emulgatoren hergestellt:

- Emulgator E1:: Mischung aus dem Natriumsalz des sulfatierten p-Octylphenolethoxylats (25 EO) und dem nicht sulfatierten p-Octylphenolethoxylates (25EO) im Mengenverhältnis 1:1.
- Emulgator E2:: Natriumsalz des sulfatierten C₁₂-Fettalkoholethoxylats (3EO). War bereits mit Formaldehyd gegen Bakterienbefall konserviert.
- Emulgator E3:: Natriumsalz des sulfatierten C₁₂-Fettalkoholethoxylats (50EO).
- Emulgator E4:: Polyethylenglykol (Molekulargewicht 9000)
- Emulgator E5:: Natriumlaurylsulfat (0 EO),Vergleichsversuch

### b. Behandlung mit Natriumpersulfat (NaPS)

Je 200 g der Emulgatorlösungen E1 bis E5 wurden unter Stickstoff auf 85 °C erhitzt, und es wurden während 2 Stunden kontinuierlich 101 g einer Lösung von 8,1 g Natriumpersulfat in 600 g Wasser zudosiert. Die Ansätze wurden eine weitere Stunde bei 85 °C gehalten. In den erhaltenen Emulgatorlösungen wurden die Formaldehydgehalte ermittelt. Die Ergebnisse zeigt Tabelle 1.

### c.Behandlung mit Wasserstoffperoxid/Ascorbinsäure(H₂O₂/Asc)

Je 200 g der Emulgatorlösungen E1 bis E5 wurden unter Stickstoff mit 1,2 g Wasserstoffperoxid (30%) versetzt und auf 60 °C erhitzt. Es wurden während 2 Stunden kontinuierlich je eine Lösung von 2,16 g Ascorbinsäure und 100 mg Eisen-II-sulfat-Heptahydrat in 100 g Wasser zudosiert und dann wurden die Ansätze 1 Stunde bei 60 °C nachgerührt. In den erhaltenen Ansätzen wurden die Formaldehydgehalte ermittelt. Die Ergebnisse zeigt Tabelle 1.

### d.Behandlung mit Azoinitiator V50 (AZO)

Je 200 g der Emulgatorlösungen E1 bis E5 wurden unter Stickstoff auf 85 °C erhitzt, und während 2 Stunden wurden kontinuierlich je 100 g einer Lösung von 9,24 g 2,2'-Azo-bis(2-amidinopropan)dihydrochlorid (V50 der Fa. Wako Chem.) in 600 g wasser zudosiert.
Die Ansätze wurden dann 1 Stunde bei 85 °C gehalten. In den erhaltenen Ansätzen wurden die Formaldehydgehalte ermittelt. Die Ergebnisse zeigt Tabelle 1.

**Tabelle 1 -**

| Formaldehydgehalt (ppm) von Initiator-behandelten Emulgatoren | | | | | |
|---|---|---|---|---|---|
| Emulgator | nEO* | unbehandelt | NaPS | H₂O₂/Asc. | AZO |
| E1 | 25 | <1 | 81 | 73 | 9 |
| E2 | 3 | 16** | 39 | 12 | 19 |
| E3 | 50 | <1 | 71 | 68 | 26 |
| E4 | 200 | 1 | 43 | 74 | 27 |
| E5 | 0 | <1 | 1 | 2 | 3 |

| | | | | | |
|---|---|---|---|---|---|
| * Gibt die ungefähre Zahl der Mole EO pro Mol Emulgator an | | | | | |
| ** Emulgator E2 enthält etwas Formaldehyd als Konservierungsmittel | | | | | |

### Beispiel 2 - Formaldehyd aus Modellverbindungslösungen

### a. Lösungen von Modellverbindungen L1 bis L4

Folgende Lösungen von Modellverbindungen wurden hergestellt:
- Lösung L1:: 3%ige Lösung von Ethylenglykol
- Lösung L2:: 3%ige Lösung von Ethylenglykoldimethylether
- Lösung L3:: 10%ige Lösung von Polyvinylalkohol (Moviol 3-83)
- Lösung L4:: 0,1%ige Lösung von n-Butylacrylat.

### b. Behandlung der Lösungen L1-L4 mit Natriumpersulfat

Die Behandlung der Lösungen L1 bis L4 erfolgte wie in Beispiel 1b angegeben. In den erhaltenen Ansätzen wurden die Formaldehydgehalte ermittelt, bei L4 der Restmonomeren-Gehalt. Die Ergebnisse zeigt Tabelle 2.

### c. Behandlung der Lösungen L1-L4 mit Wasserstoffperoxid/Ascorbinsäure (H₂O₂/Asc.)

Die Behandlung der Lösungen L1-L4 erfolgte wie in Beispiel 1c angegeben. In den erhaltenen Ansätzen wurden die Formaldehydgehalte ermittelt, bei L4 der Restmonomeren-Gehalt. Die Ergebnisse zeigt Tabelle 2.

### d. Behandlung der Lösungen L1-L4 mit tert.Butylhydroperoxid und Ascorbinsäure (tBHP/Asc.)

Zu 200 g der Lösungen L1 bis L4 wurden bei 60 °C je 1,0 g tert.-Butylhydroperoxid (70%ig) und 200 g einer Lösung von 2,16 g Ascorbinsäure und 10 mg Eisen-II-sulfat-Heptahydrat in 600 g Wasser zudosiert und dann jeweils 1 Stunde nachgerührt. In den erhaltenen Ansätzen wurden die Formaldehydgehalte ermittelt, bei L4 der Restmonomerengehalt. Die Ergebnisse zeigt Tabelle 2.

### e. Behandlung einer n-Butylacrylat-Lösung

Entsprechend den Behandlungen der Lösungen L1 bis L3 wurden 400 g der n-Butylacrylat-Lösung behandelt und jeweils der Restmonomerengehalt durch Gaschromatographie bestimmt. Er lag stets <10 ppm und beweist damit, daß die Versuche unter Polymerisationsbedingungen ausgeführt wurden.

**Tabelle 2 -**

| Formaldehydgehalt bei Initiator-behandelten Modellverbindungen | | | |
|---|---|---|---|
| Modellverbindung | NaPS | H₂O₂/Asc. | tBHP/Asc. |
| L1-Ethylenglykol | 37 | 22 | 7 |
| L2-Ethylenglykol-dimethylether | 106 | 121 | 25 |
| L3-Polyvinyl-alkohol | 3 | 9 | 10 |
| L4-n-Butylacrylat | <10* | <10* | <10* |

| | | | |
|---|---|---|---|
| * Restmonomerengehalt an n-Butylacrylat | | | |

### Beispiel 3 - Zusatz verschiedener Mengen an Wasserstoffperoxid zu ethoxyliertem Emulgator

Zu jeweils 200 g einer 3 %igen Lösung des Natriumsalzes eines C₁₂-Fettalkoholpolyethylenoxidsulfats (ca.30 EO) als Emulgator wurden 60 g unterschiedlich konzentrierte Lösungen von Wasserstoffperoxid in Wasser gegeben und auf 60 °C aufgeheizt. Ascorbinsäure (2,5 fache Gewichtsmenge von Wasserstoffperoxid) und 5 Tropfen einer 1 %igen Eisen-II-sul-fatlösung wurden mit Wasser auf 60 g Lösung verdünnt und während 2 Stunden der Emulgatorlösung zugegeben. Nach dem Abkühlen wurden in den Ansätzen jeweils die entstandenen Mengen an Formaldehyd, Acetaldehyd und Glyoxal (in ppm) nach Methode 2 bestimmt. Die Ergebnisse zeigt Tabelle 3.

**Tabelle 3 -**

| Aldehydgehalt in Abhängigkeit der Menge an Wasserstoffperoxid | | | | |
|---|---|---|---|---|
| Beisp. | Mengenverhältnis H₂O₂ zu Emulgator | Formaldehyd ppm | Acetaldehyd ppm | Glyoxal ppm |
| 3a | 0 | 1 | <0,5 | <0,5 |
| 3b | 0,1 | 3 | 0,5 | 0,5 |
| 3c | 0,2 | 6 | 3 | 4 |
| 3d | 0,3 | 5 | 0,8 | 2 |
| 3e | 0,5 | 11 | 2 | 9 |
| 3f | 1,0 | 21 | 5 | 15 |
| 3g | 1,0* | 61(113) | 2(5) | 32(4) |
| 3h | 5,0 | 77 | 102 | 106 |
| 3i | 10 | 22 | 116 | 100 |
| 3k | 1,0** | 6 | 2 | 3 |

| | | | | |
|---|---|---|---|---|
| * Bei Versuch 3g wurde der Formaldehyd-Wert vor der Reaktion mit 4 Tropfen Formaldehydlösung erhöht. In Klammern sind die Aldehydwerte vor der Zugabe des Reduktionsmittels angeführt. Die Abnahme des Formaldehyd-Wertes ist zu etwa gleichen Teilen auf die verdünnung und die Oxydation zurückzuführen. | | | | |
| ** Bei Versuch 3k wurde die Emulgatorlösung durch reines Wasser ersetzt. Die Aldehyde entstehen nicht aus der Ascorbinsäure. | | | | |

### Beispiel 4 - Vergleich Emulgator mit Blockcopolymer

Jeweils 200 ml von 3 %igen wässrigen Lösungen des Natriumsalzes eines C₁₂-Fettalkoholpolyethylenoxidsulfats (ca.30 EO) (EMU-4) und eines Polystyrol-Polyethylenoxid-Blockcopolymeren mit einem mittleren Polymerisationsgrad von 10 bzw. 70 (SE 1030 der Fa.Th.Goldschmidt) wurden bei 85 °C während 2 Stunden mit einer wässrigen Lösung von 8,1 g Natriumpersulfat in 600 g Wasser behandelt. Dann wurde der Ansatz eine Stunde gerührt und abgekühlt. Es wurde der Gehalt an Formaldehyd und Acetaldehyd nach Methode 2 bestimmt.
Die Ergebnisse zeigt Tabelle 4.

**Tabelle 4 -**

| Behandlung von Emulgator und Blockcopolymer | | | |
|---|---|---|---|
| Emulgator | Behandlung mit | Formaldehyd ppm | Acetaldehyd ppm |
| EMU-4 | unbehandelt | <5 | <5 |
| PS-PEO-Block | unbehandelt | <5 | <5 |
| EMU-4 | Persulfat | 150 | 500 |
| PS-PEO-Block | Persulfat | 50 | 100 |

### Beispiel 5 - Absenkung von Glyoxal

50 ml einer 1 molaren wäßrigen Lösung von Glyoxal wurde mit jeweils 50 ml unterschiedlich konzentrierter Lösungen von Harnstoff und Ethylenharnstoff (0,5 M, 1 M, 2 M), die in 5 %iger Kaliumhydrogensulfatlösung gelöst sind, vermischt und 24 Stunden bei 25 °C stehen gelassen. Danach wurde in der Mischung der Gehalt an freiem Glyoxal nach Methode 2 bestimmt. Die Bestimmung erfolgte durch Abtrennung von Glyoxal per HPLC auf einer RP-Säule mit nachfolgender Derivatisierung mittels Acetylaceton. Das entstandene Lutidin-Derivat wurde mittels UV-Detektion nach der Methode des externen Standards quantifiziert. Tabelle 5 gibt die wiedergefundene Prozentmenge an eingesetztem Glyoxal an.

**Tabelle 5 -**

| Glyoxal-Bestimmung | |
|---|---|
| Zusatz von | %- Menge an wiedergefundenem Glyoxal |
| Wasser | 100 |
| 0,5 M Harnstoff | 87 |
| 1 M Harnstoff | 80 |
| 2 M Harnstoff | 61 |
| 0,5 M Ethylenharnstoff | 100 |
| 1 M Ethylenharnstoff | 92 |
| 2 M Ethylenharnstoff | 83 |

### Beispiel 6

Eine Monomerenemulsion ME6 wurde aus 6,29 kg Wasser, 0,90 kg eines Natriumsalzes des sulfatierten C₁₂-Fettalkohols (50 EO), 13,0 kg Ethylacrylat und 0,50 kg Acrylamidoglykolsäure hergestellt.

Eine Vorlage aus 7,17 kg Wasser und 54 g Wasserstoffperoxid (50%) wurde unter Stickstoff auf 60 °C aufgeheizt und mit 400 g der Monomerenemulsion ME6 und 100 g einer Lösung von 27 g Ascorbinsäure und 0,15 g Eisen-II-sulfat in 2 kg Wasser versetzt und 15 Minuten gerührt. Der Rest der Monomerenemulsion ME6 wurde in 120 Minuten, der Rest der Reduktionsmittellösung in 135 Minuten kontinuierlich zudosiert. Nach dem Abkühlen wurde der Ansatz mit 20 g einer 10%igen wässrigen Lösung von tert.Butylhydroperoxid und 13 g einer 10%igen Lösung von Ascorbinsäure versetzt. Nach Probenahme wurde eine Lösung von 13,5 g Ethylenharnstoff in 54 g Wasser zugegeben und 1 Stunde bei 25 °C gerührt. Es wurde eine niedrigviskose Dispersion (16 mPas bei 480/s) mit einem Feststoffgehalt von 44,2% und einer Lichtdurchlässigkeit von 68% erhalten. Der Koagulatanteil betrug 0,13 %. Der Formaldehydanteil betrug vor der Zugabe von Ethylenharnstoff 11 ppm, nach der Zugabe wurde er mit <1 ppm bestimmt.

### Beispiel 7

Beispiel 6 wurde wiederholt, jedoch wurde anstelle des Ethylenharnstoffs Harnstoff in einer Menge von 67,5 g verwendet. Die Formaldehydmenge nahm von 20 ppm auf <1 ppm ab.

### Beispiel 8

4 Monomerenansätze aus je 650 g Wasser, 720 g n-Butylacrylat, 456 g Methylmethacrylat und 24 g Acrylsäure wurden unter Zugabe der in Tabelle 6 angegebenen Emulgatoren A bis F in 4 Monomerenemulsionen ME-8A bis ME-8D überführt:
- Emulgator A:: Natriumsalz eines sulfatierten C₁₂-Fettalkoholethoxylats (50 EO) 30 %ig.
- Emulgator B:: C₁₆-C₁₈-Fettalkoholethoxylat (30 EO) 20 %ig.
- Emulgator C:: xNatriumsalz des sulfatierten p-Octylphenolethoxylats (25 EO) 35 %ig.
- Emulgator D:: p-Octylphenolethoxylat (25 EO) 20 %ig.
- Emulgator E:: Polyethylenoxid Molekulargewicht 200 g/Mol
- Emulgator F:: Natriumlaurylsulfat 15 %ig.

Jeweils 880 g Wasser wurden unter Stickstoff auf 85 °C aufgeheizt und jeweils mit 38 g von einer der Monomerenemulsionen ME-8A bis ME-8D und 12 g einer Initiatorlösung von 8 g Natriumpersulfat in 234 g Wasser (Initiatorlösung I8) versetzt und 15 Minuten lang gerührt. In die bei 85 °C gehaltenen Ansätze wurde jeweils der Rest der Monomerenemulsionen ME-8A bis ME-8D während 120 Minuten und der Rest der Initiatorlösungen I8 kontinuierlich zudosiert und danach je 1 Stunde weitergerührt. Nach dem Abkühlen wurden jedem Ansatz 12 g tert.Butylperoxid und 12 g Ascorbinsäure (je in Form von 10%igen wässrigen Lösungen) zugesetzt. In den resultierenden 4 koagulatfreien Polymerdispersionen wurden jeweils die Formaldehydgehalte nach Methode 1 bestimmt, ferner die Feststoffgehalte, die Lichtdurchlässigkeiten (LD), die pH-Werte sowie wie oben angegeben die Schaumbildung. Die in Tabelle 6 angegebenen Ergebnisse zeigen, daß der Vergleichsversuch d mit Natriumlaurylsulfat deutlich weniger Formaldehyd freisetzt und zu stärkerer Schaumbildung führt.

**Tabelle 6 -**

| Bildung von Formaldehyd und Schaum bei verschiedenen Emulgatoren | | | | |
|---|---|---|---|---|
| Versuch | a | b | c | d |
| Emulgator-menge/-art | 40 g A +30 g B | 52 g C +60 g D | 3,6 g E + 56 g F | 80 g F (Vgl.) |
| Feststoff-gehalt % | 39,4 | 39,6 | 39,4 | 39,7 |
| pH-Wert | 2,0 | 1,9 | 1,8 | 1,9 |
| LD-Wert % | 46 | 33 | 28 | 38 |
| Schaumbildung | 170 | 130 | 200 | 650 |
| Formaldehyd ppm | 21 | 8 | 9 | 4 |

## Patentansprüche

1. Verfahren zur Verminderung eines unter Radikaleinwirkung aus organischen Verbindungen mit -CH₂-CHR-O- und/oder - CH₂-CH(OH)-Gruppierungen (R = H, C₁-C₄-Alkyl) gebildeten Gehalts an C₁-C₂-Aldehyden in Substanzen, Dispersionen oder Lösungen, die solche Gruppierungen oder Verbindungen mit solchen Gruppierungen enthalten und die keine zugesetzten Verbindungen mit Aldehydderivat-Gruppen aufweisen, die bekannterweise C₁-C₂-Aldehyde freisetzen können, durch Zusatz von mit den Aldehydgruppen reagierenden stickstoffhattigen Verbindungen, welche den Aldehyd binden und die ausgewählt und unter Harnstoff, Harnstoff-Derivaten, Copolymeren, die Monomere mit cyclischen Harnstoffgruppen ein polymerisiert enthalten; wasserläslichen Copolymeren mit Amidgruppen; Carbamaten und cyclischen Verbindungen mit NH-Gruppen.

2. Verfahren nach Anspruch 1, **dadurch gekennzeichnet, daß** man als stickstoffhaltige Verbindungen Harnstoff und/oder Harnstoffderivate zusetzt.

3. Verfahren nach Anspruch 1, **dadurch gekennzeichnet, daß** die verfahrensgemäß zu behandelnden Substanzen, Dispersionen oder Lösungen als organische Verbindungen mit -CH₂-CHR-O-Gruppierungen C₁-C₄-Alkylenglykole, Poly-C₁-C₄-alkylenglykole oder Derivate derselben enthalten.

4. Verfahren nach Anspruch 1, **dadurch gekennzeichnet, daß** die verfahrensgemäß zu behandelnden Substanzen, Dispersionen oder Lösungen als organische Verbindungen mit -CH₂-CH(OH)-Gruppierungen Polyvinylalkohole enthalten.

5. Verfahren nach Anspruch 1, **dadurch gekennzeichnet, daß** die verfahrensgemäß zu behandelnden Substanzen, Dispersionen oder Lösungen als organische Verbindungen mit -CH₂-CHR-O- und/oder - CH₂-CH(OH)-Grugpierungen solche enthalten, die als Emulgatoren und/oder Schutzkolloide bei der Herstellung und/oder Verwendung von wässrigen Polymerdispersionen wirken.

6. Verfahren nach Anspruch 1, **dadurch gekennzeichnet, daß** verfahrensgemäß wässrige Polymerdispersionen oder -lösungen behandelt werden, die durch radikalische Polymerisation von olefinisch ungesättigten Monomeren in Gegenwart von organischen Verbindungen mit -CH₂-CHR-O- und/oder -CH₂-CH(OH)-Gruppierungen (R = H, C₁-C₄-Alkyl) hergestellt sind.

7. Verfahren nach Anspruch 1, **dadurch gekennzeichnet, daß** die verfahrensgemäß behandelten Substanzen, Dispersionen oder Lösungen keine Monomeren, Monomereinheiten, Initiatoren und/oder Vernetzer mit N-Methylolgruppen und/oder Ethern oder Estern solcher Gruppen enthalten.

## Claims

1. A process for reducing a content of C₁-C₂-aldehydes formed, by the action of free radicals, from organic compounds containing -CH₂-CHR-O- and/or -CH₂-CH(OH)- groups (R = H, C₁-C₄-alkyl), in materials, dispersions or solutions which contain such groups or compounds with such groups and which do not have added compounds containing aldehyde derivative groups which are known to be able to release C₁-C₂-aldehydes, by adding nitrogen-containing compounds which react with the aldehyde groups, which bind the aldehyde and which are selected from urea; urea derivatives; copolymers which contain monomers with cyclic urea groups in copolymerized form; water-soluble copolymers with amide groups; carbamates and cyclic compounds with NH groups.

2. A process as claimed in claim 1, wherein the nitrogen-containing compounds are urea and/or urea derivatives.

3. A process as claimed in claim 1, wherein the materials, dispersions or solutions to be treated according to the process comprise, as organic compounds containing -CH₂-CHR-O-groups, C₁-C₄-alkylene glycols, poly-C₁-C₄-alkylene glycols or derivatives thereof.

4. A process as claimed in claim 1, wherein the materials, dispersions or solutions to be treated according to the process comprise, as organic compounds containing - CH₂-CH(OH)- groups, polyvinyl alcohols.

5. A process as claimed in claim 1, wherein the organic compounds containing -CH₂-CHR-O- and/or -CH₂-CH(OH)- groups in the materials, dispersions or solutions to be treated according to the process comprise, are those which act as emulsifiers and/or protective colloids during the preparation and/or use of aqueous polymer dispersions.

6. A process as claimed in claim 1, wherein, according to the process, aqueous polymer dispersions or solutions are treated which have been prepared by free-radical polymerization of olefinically unsaturated monomers in the presence of organic compounds containing -CH₂-CHR-O- and/or -CH₂-CH(OH)- groups (R = H, C₁-C₄-alkyl).

7. A process as claimed in claim 1, wherein the materials, dispersions or solutions treated according to the process do not contain monomers, monomer units, initiators and/or crosslinkers containing N-methylol groups and/or ethers or esters of such groups.

## Revendications

1. Procédé pour réduire une teneur en aldéhydes en C₁ à C₂ formée par l'action de radicaux à partir de composés organiques comportant des groupes -CH₂-CHR-O- et/ou -CH₂-CH(OH) (R = H, alkyle en C₁ à C₄), dans des substances, des dispersions ou des solutions contenant ces groupes ou des composés comportant ces groupes, et ne présentant pas de composés additionnels à groupes dérivés d'aldéhydes pouvant libérer de manière en soi connue des aldéhydes en C₁ à C₂, par addition de composés azotés réagissant avec les groupes aldéhyde, qui lient les groupes aldéhyde et sont choisis parmi l'urée, des dérivés d'urée, des copolymères contenant des monomères à groupes urée cycliques copolymérisés, des copolymères solubles dans l'eau comportant des groupes amide, des carbamates et des composés cycliques comportant des groupes OH.

2. Procédé selon la revendication 1, **caractérisé en ce que** l'on ajoute en tant que composés azotés de l'urée et/ou des dérivés d'urée.

3. Procédé selon la revendication 1, **caractérisé en ce que** les substances, dispersions ou solutions traitées par le procédé contiennent en tant que composés organiques à groupes -CH₂-CHR-O des (C₁-C₄)alkylène glycols, des poly(C₁-C₄)alkylène glycols ou des dérivés de ceux-ci.

4. Procédé selon la revendication 1, **caractérisé en ce que** les substances, dispersions ou solutions à traiter par le procédé contiennent en tant que composés inorganiques à groupes -CH₂-CH(OH) des poly(alcools vinyliques).

5. Procédé selon la revendication 1, **caractérisé en ce que** les substances, dispersions ou solutions à traiter par le procédé contiennent en tant que composés organiques à groupes -CH₂-CHR-O-et/ou -CH₂-CH(OH) ceux qui agissent comme émulsifiants et/ou colloïdes protecteurs lors de la préparation et/ou de l'utilisation de dispersions aqueuses de polymères.

6. Procédé selon la revendication 1, **caractérisé en ce que** l'on traite par le procédé des dispersions ou des solutions aqueuses de polymères, préparées par polymérisation radicalaire de monomères oléfiniquement insaturés en présence de composés organiques comportant des groupes -CH₂-CHR-O- et/ou -CH₂-CH(OH) (R = H, alkyle en C₁ à C₄).

7. Procédé selon la revendication 1, **caractérisé en ce que** les substances, dispersions et/ou solutions traitées par le procédé ne contiennent pas de monomères, d'unités monomères, d'initiateurs et/ou de réticulants comportant des groupes N-méthylol et/ou d'éthers ou d'esters de ces groupes.
